# EUROPEAN PATENT APPLICATION

(11) **EP 1 724 295 A1**
(43) Date of publication of application: **22.11.2006**
(21) Application number: 05720406.7
(22) Date of filing: 09.03.2005
(51) Int. Cl.: C08G 85/00, B01J 31/28, C01B 3/04, C07D 471/06, C07D 519/00

(54) **SUPRAMOLECULAR COMPLEX OF PYRIDYLNAPHTHALENEDIIMIDE WITH ZINC PORPHYRIN DENDRIMER HAVING MULTIPLICITY OF ARTIFICIAL PHOTOSYNTHETIC REACTION CENTER**

(30) Priority: 10.03.2004 JP 2004068248
(71) Applicant: Japan Science and Technology Agency, Kawaguchi-shi, Saitama 332-0012 (JP)
(72) Inventor: FUKUZUMI, Shunichi, Osaka 5620031 (JP)
(74) Representative: UEXKÜLL & STOLBERG
(86) International application number: PCT/JP2005/004135
(87) International publication number: WO 2005/087846

(57) **Abstract**

A light energy conversion system having a multiplicity of artificial photosynthetic reaction centers and exhibiting a high light-harvesting capability and charge separation capability, constructed by the use of a zinc porphyrin dendrimer supramolecular complex formed by the use of a coordinate bond. Using a zinc porphyrin dendrimer as a spherical zinc porphyrin oligomer, a supramolecule with pyridylnaphthalenediimide was constructed. With respect to the zinc porphyrin dendrimer/pyridylnaphthalenediimide supramolecule, when photoexcitation of the zinc porphyrin was realized, a charge-separated state was generated by photoinduced electron transfer and the lifetime thereof was strikingly long, up to 830 microseconds.

## Description

### TECHNICAL FIELD

The present invention relates to a zinc porphyrin dendrimer-pyridylnaphthalenediimide supramolecular complex having numerous artificial photosynthetic reaction centers.

### BACKGROUND ART

In natural photosynthesis, one reaction center is associated with a number of light harvesting units in which the harvested light energy is efficiently transferred to the reaction center.

Conventionally, numerous artificial photosynthesis model systems have been studied that mimic the energy and electron transfer process in such natural photosynthesis. However, mainly artificial photosynthetic reaction center molecules in which the electron donor molecule and the electron acceptor molecule are linked by a covalent bond have been studied. Forexample, J. Phys. Chem. A2002, 106, 3243-3252 (non-patent document 1) discloses a compound in which porphyrin and fullerene are linked by a covalent bond.

However, regarding molecules in which an electron donor molecule and an electron acceptor molecule are linked as an artificial photosynthetic reaction center molecule, there are limits to the light-harvesting function thereof. In other words, there is the disadvantage that these materials cannot have both a high light-harvesting function and a charge separation function at the same time.

On the other hand, in order to enhance the light-harvesting capability of an artificial photosynthetic reaction center, integration of porphyrin molecules has been carried out by various methods. However, none has integrated reaction centers having a charge separation function. Artificial photosynthetic model systems having numerous reaction centers have not yet been developed, due to the difficulty of synthesizing dendritic donor-acceptor arrays. For this reason, molecules having both a high light-harvesting function and a charge separation function at the same time have yet to be developed.
Non-patent document 1: J. Phys. Chem. A 2002, 106, 3243-3252

### DISCLOSURE OF THE INVENTION

### PROBLEMS TO BE SOLVED BY THE INVENTION

Hereafter, the present invention will be described in detail.

An object of the present invention is to construct a light energy conversion system having numerous artificial photosynthetic reaction centers and exhibiting a high light-harvesting capability and a charge separation capability by using a zinc porphyrin dendrimer supramolecular complex that is formed by using a coordination bond.

### MEANS FOR SOLVING THE PROBLEMS

As a result of eager studies, the present inventors have succeeded in constructing a multiple photosynthesis reaction center by using a supramolecular complex formed between a zinc porphyrin dendrimer [D(ZnP)₁₆] (containing sixteen zinc porphyrins) and a pyridylnaphthalenediimide (PyNIm) (having a bonding site (pyridyl moiety) to zinc ions of (D(ZnP)₁₆) in benzonitrile. The apparent formation constant determined from the fluorescence quenching in a singlet excited state of zinc porphyrins by PyNIm was considerably larger than the formation constant determined by the UV-vis spectra change caused by the formation of a supramolecular complex. This shows that an efficient energy migration between zinc porphyrin moieties is taking place, and causes the fluorescence quenching in a singlet excited state of zinc porphyrins by PyNIm that is bonded to a different zinc porphyrin moiety. The charge-separated (CS) state of this supramolecular dendrimer complex was detected as a transient absorption spectrum in laser flash photolysis, with a result that the CS state produced at the time of laser excitation had a long lifetime (830 µs) at 298K in PhCN.

Specifically, the present invention provides the following supramolecular complex and the like.

(1) A complex compound containing a porphyrin dendrimer and a substituted or non-substituted pyridylnaphthalenediimide, wherein a metal is bonded to porphyrins in the compound, and a pyridyl group of the pyridylnaphthalenediimide is axially coordinated to the metal.

(2) The compound according to item 1, wherein said metal is zinc.

(3) A complex compound represented by the following formula 1:

(Chemical formula 1) R¹(-N(-R²-N(-R³-N(-R⁴-NHCO-R⁵-CONH-R⁶-R⁷)₂)₂)₂)₂

wherein R¹ is alkylene; four R² are independently alkylene; eight R³ are independently alkylene; sixteen R⁴ are independently alkylene; sixteen R⁵ are independently alkylene; sixteen R⁶ are independently a porphyrin having a metal; the metal is bonded to a central part of each porphyrin; sixteen R⁷ are independently a substituted or non-substituted pyridylnaphthalenediimide; and a pyridyl group of the substituted or non-substituted pyridylnaphthalenediimide is axially coordinated to each of the sixteen metal atoms.

(4) The compound according to item 3, wherein said substituted or non-substituted pyridylnaphthalenediimide is N-pyridyl-N'-alkyl-naphthalenediimide, and the alkyl group has a carbon number of 3 to 9.

(5) The compound according to item 3, wherein R¹, four R², eight R³, sixteen R⁴, and sixteen R⁵ are all -C₃H₆- .

(6) The compound according to item 3, wherein said sixteen metals are all zinc.

(7) The compound according to item 3, wherein said sixteen porphyrins each have a phenyl group, an alkyl-substituted phenyl group, or a halogen-substituted phenyl group at 5-position, 10-position, 15-position, and 20-position thereof.

(8) The compound according to item 3, wherein said sixteen porphyrins each have a phenyl group, a 3,5-di-*tert*-butylphenyl group, or a 2,6-dichlorophenyl group at 5-position, 10-position, 15-position, and 20-position thereof.

(9) A method of synthesizing the compound of item 1, comprising a step of mixing said porphyrin dendrimer and a substituted or non-substituted pyridylnaphthalenediimide in a solvent.

(10) A method of synthesizing the compound of item 3, comprising a step of mixing a porphyrin dendrimer of the following formula and a substituted or non-substituted pyridylnaphthalenediimide in a solvent:

(Chemical formula 2) R¹(-N(-R²-N(-R³-N(-R⁴-NHCO-R⁵-CONH-R⁶)₂)₂)₂)₂

(11) The method according to item 10, wherein said solvent is benzonitrile.

(12) A material for an artificial photosynthetic reaction center made of the compound according to item 1.

(13) A hydrogen-producing photocatalyst containing the compound according to item 1 and a platinum catalyst.

(14) A method of synthesizing hydrogen, comprising a step of irradiating water with light in the presence of a molecule serving as an electron source and a hydrogen-producing photocatalyst containing the supramolecular complex according to item 1 and a platinum catalyst.

(15) A device for converting light into electric current, wherein the compound according to item 1 is laminated on an electroconductive substrate.

### EFFECTS OF THE INVENTION

In the present invention, in order to outstandingly improve the light-harvesting capability, a supramolecular complex is formed by using a zinc porphyrin dendrimer, forming a coordination bond between the zinc porphyrin dendrimer and a pyridylnaphthalenediimide. Thus, a light energy conversion system having numerous artificial photosynthetic reaction centers exhibiting high light-harvesting capability and a charge separation capability, is constructed. There are no other examples of such light energy conversion supramolecule having both high light-harvesting capability and charge separation capability.

### BRIEF DESCRIPTION OF THE DRAWINGS

[Figure 1]

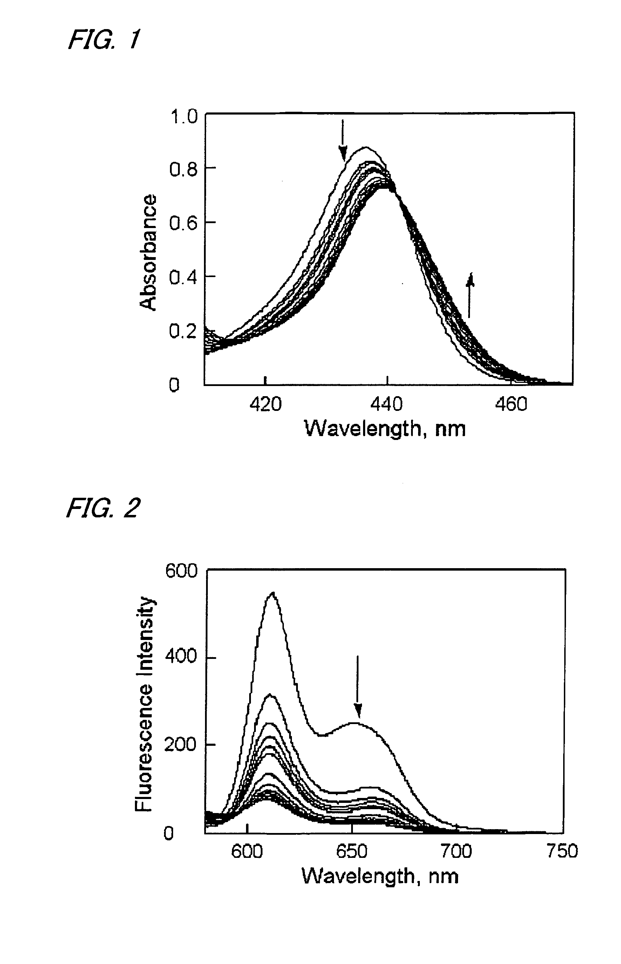

   Figure 1 shows a UV-vis spectral changes of D(ZnP)₁₆ (2.9 x 10⁻⁶ M based on the number of porphyrin units) in the presence of various concentrations of PyNIm (0 to 4.9 mM) in PhCN at 298K.
[Figure 2]
   Figure 2 shows a fluorescence spectra of D(ZnP)₁₆ (2.9 x 10⁻⁶ M) at 298K in deaerated PhCN in the presence of various concentrations of PyNIm (0 to 4.9 mM).
[Figure 3]

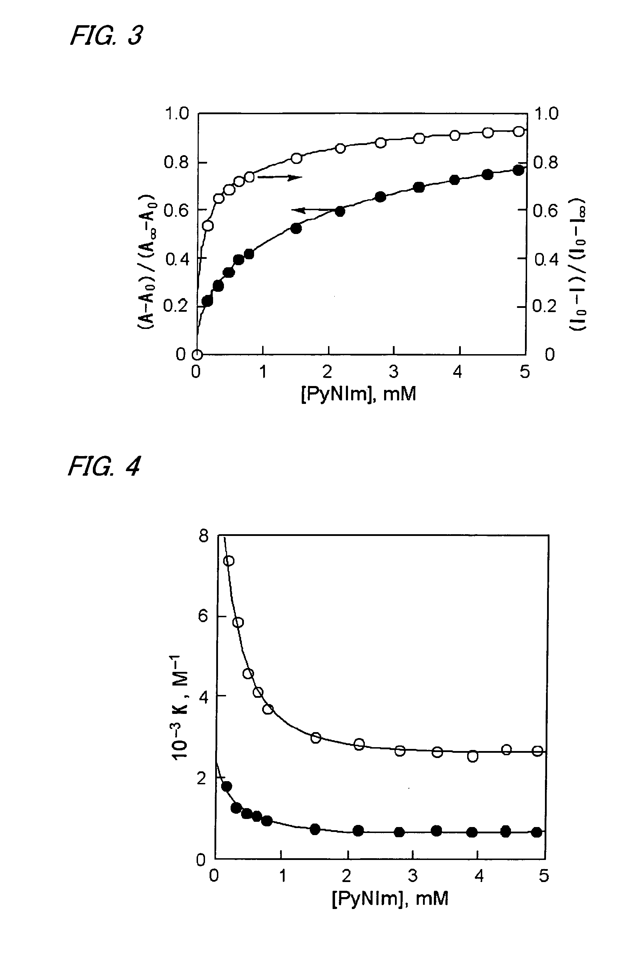

   Figure 3 shows plots of (A - A₀) / (A₈ - A₀) (closed circles) and (I₀ - I) / (I₀ - I₈) (open circles) relative to [PyNIm]. (A₈ - A₀) and (I₀ - I₈) are estimated by extrapolation of [PyNIm] dependence of spectral changes (fluorescence intensity at 659 nm and absorbance at 450 nm). (A₈ - A₀) = 0.195, (I₀ - I₈) = 324.
[Figure 4]
   Figure 4 shows dependence of K of supramolecular complex formation between the ZnP moieties of D(ZnP)₁₆ with PyNIm at 298K in PhCN, relative to the concentration of PyNIm. The closed circles and the open circles correspond to the K values determined from the plots of (A - A₀) / (A₈ - A₀) and (I₀ - I) / (I₀ - I₈), respectively.
[Figure 5]

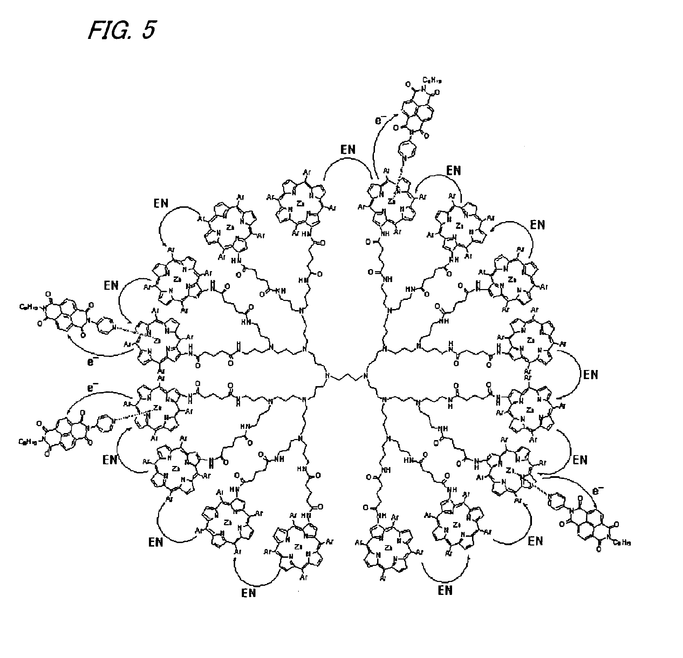

   Figure 5 shows a schematic presentation of energy migration of the singlet excited state and electron transfer in the supramolecular D(ZnP-PyNIm)₁₆ complex. In this figure, EN refers to energy migration.
[Figure 6]

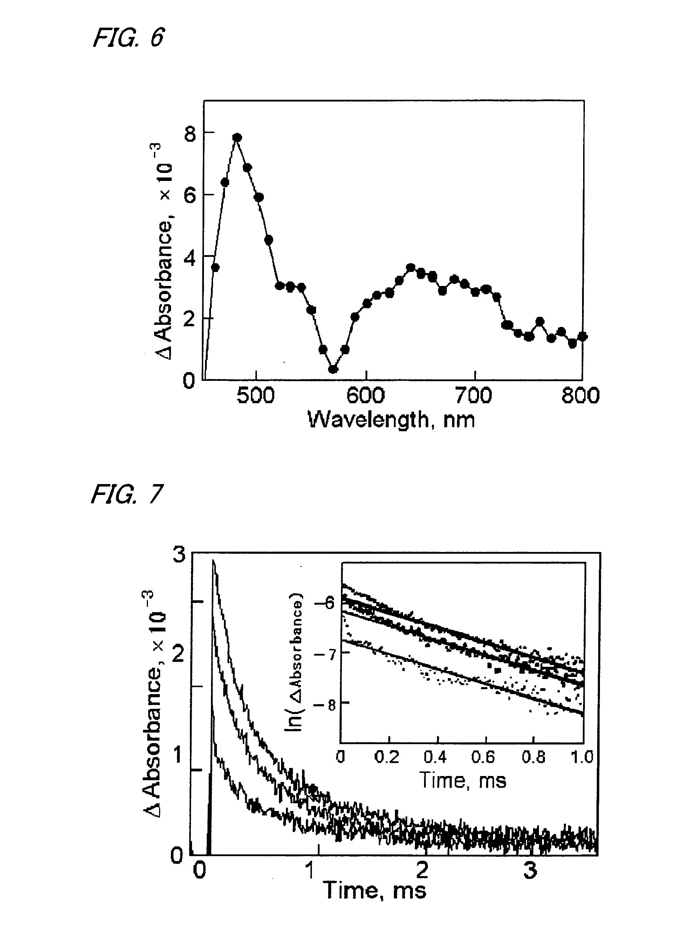

   Figure 6 shows a transient spectrum of D(ZnP)₁₆ (2.9 x 10⁻⁶ M) at 298K in deaerated PhCN in the presence of PyNIm (4.9 x 10⁻³ M), as measured at 20 µs after the laser excitation at 431 nm.
[Figure 7]
   Figure 7 shows a time profile of absorption at 620 nm due to the CS state obtained upon nanosecond flash photolysis with different laser power (10 mJ, 2.2 mJ, and 0.5 mJ, respectively). The inset shows the first-order plot for the decay of absorbance at620 nm (10 mJ, crosses; 2.2 mJ, closed squares; 0.5 mJ, closed circles).

### BEST MODE FOR CARRYING OUT THE INVENTION

Hereafter, embodiments of the present invention will be described more specifically.

### (Porphyrin)

The porphyrin used in the present invention may be a non-substituted porphyrin or may be one having arbitrary substituents within a scope that does not deteriorate its performance as a porphyrin. For example, the porphyrin may have, as a substituent, an alkyl, an aryl, an alkyl-substituted aryl, or a halogen-substituted aryl at 5-position, 10-position, 15-position, and 20-position in the porphyrin, in other words, at the carbon atom of the methine group between a pyrrole ring and a pyrrole ring. This substituent is preferably a phenyl or a substituted phenyl, morepreferablyaphenyl, analkyl-substituted phenyl, or a halogen-substituted phenyl, still more preferably a phenyl, a dialkyl-substituted phenyl, or a dichloro-substituted phenyl, particularly preferably a phenyl, a 3,5-di-*tert*-butylphenyl, or 2,6-dichlorophenyl. It is noted that the 5-position, the 10-position, the 15-position, and the 20-position of the porphyrin preferably all have the same substituent; however, two to four kinds of substituents may be introduced to the four positions of 5-position, 10-position, 15-position, and 20-position if necessary.

A preferred example of porphyrin is shown below. 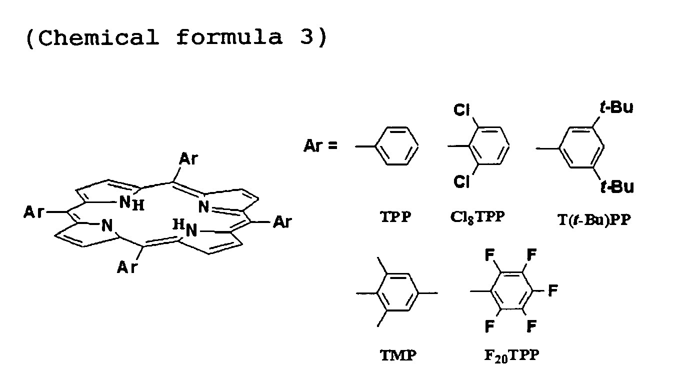 Further, if necessary, in the porphyrin, a substituent may be introduced to the pyrrole rings within a range that does not deteriorate the performance of the porphyrin.

In this regard, when an alkyl is introduced as a substituent to any position of the porphyrin, the carbon number thereof is not particularly limited; however, the carbon number is preferably 1 to 10, more preferably 1 to 6.

### (Porphyrin dendrimer)

In the present specification, a porphyrin dendrimer refers to a compound in which a plurality of porphyrins form a dendrimer structure (tree-shaped structure) in one molecule. Preferably the number of porphyrins in a dendrimer is two or more, more preferably 4 or more, still more preferably 8 or more, and most preferably 16 or more. Also, although there is no particular upper limit to the number of porphyrins in a molecule, the number of porphyrins is 64 or less in one embodiment, and is 32 or less in another embodiment. In one particularly preferable embodiment, the number of porphyrins in one molecule is 16.

In one preferable embodiment, the porphyrin dendrimer has the following structure.

(Chemical formula 2) R¹(-N(-R²-N(-R³-N(-R⁴-NHCO-R⁵-CONH-R⁶)₂)₂)₂)₂

In the formula, R¹, R², R³, R⁴, and R⁵ are each independently an alkylene, and the carbon number thereof is preferably 1 to 10, more preferably 2 to 8, and still more preferably 3 to 6. In one preferable embodiment, the carbon number of the alkylene is 3. R¹, R², R³, R⁴, and R⁵ may all be the same or may be different from each other; however, it is preferable to set all of them to be the same in view of the facility in producing the dendrimer. In particular, it is preferable to set all of R², R³, R⁴ , and R⁵ to be the same. In one preferable embodiment, R¹, R², R³, R⁴, and R⁵ can all be -C₃H₆-. Also, in another preferable embodiment, it is possible to set only R¹ to be -C₄H₈-, and all of R², R³, R⁴, and R⁵ to be -C₃H₆-.

Also, the alkylene may be a straight chain or may be a branched chain.

In the above formula, R⁶ are independently a porphyrin having a metal, and the metal is bonded to the central part of each porphyrin.

### (Metal)

In the present invention, the metal that is to be coordinated to the central moiety of the porphyrin can be any metal atom that can be bonded to the central moiety of the porphyrin and can form a coordination bond with pyridylnaphthalenediimide. The metal is preferably a transition metal, more preferably zinc.

### (Pyridylnaphthalenediimide)

In the present invention, a substituted or non-substituted pyridylnaphthalenediimide is used. The general formula is shown below. Please note that, in the present specification, for the sake of simplifying the description, in some cases, a substituted or non-substituted pyridylnaphthalenediimide are simply referred to as "pyridylnaphthalenediimide". 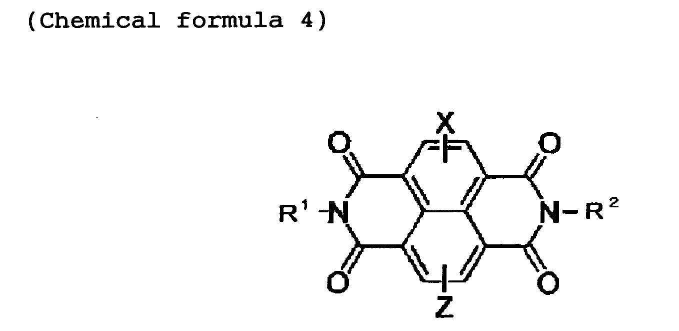 In the chemical formula 4, R¹ is a substituted or non-substituted pyridyl, preferably a non-substituted pyridyl. With regard to the positional relationship with naphthalenediimide, the pyridyl may be any of 2-pyridyl, 3-pyridyl, and 4-pyridyl. In one preferable embodiment, the pyridyl is 4-pyridyl. When the pyridyl has a substituent, the substituent is selected within a range that does not deteriorate the performance of the pyridyl. For example, the substituent can be an alkyl or a halogen. Please note that the carbon number of the alkyl is not particularly limited. However, the carbon number is preferably 1 to 10. The alkyl may be a straight chain or a branched chain.

R² is hydrogen or an alkyl. Preferably, R² is an alkyl having a carbon number of 1 to 20, more preferably an alkyl having a carbon number of 2 to 15, still more preferably an alkyl having a carbon number of 3 to 9, particularly preferably an alkyl having a carbon number of 4 to 8, and in one preferable embodiment, R² is an alkyl having a carbon number of 6. Further, the alkyl may be a straight chain or a branched chain.

X and Z are independently hydrogen, an alkyl, or a halogen, preferably hydrogen. In this regard, the carbon number of the alkyl is not particularly limited; however, the carbon number is preferably 1 to 10, more preferably 1 to 6. Further, the alkyl may be a straight chain or a branched chain. When X is other than hydrogen, the number thereof may be 1 or 2. When Y is other than hydrogen, the number thereof may be 1 or 2.

The pyridylnaphthalenediimide to be used in the present invention may be a non-substituted pyridylnaphthalenediimide or may be a substituted pyridylnaphthalenediimide. An example of the substituent in a substituted pyridylnaphthalenediimide compound may be an alkyl, though the substituent is not particularly limited. The number of the substituents is not particularly limited. Preferably, the number of alkyl substituents is 1. The length of the chain in the alkyl substituent to be used is not particularly limited. In one embodiment, the carbon number in the alkyl substituent is 3 or more, and in a more preferable embodiment, the carbon number is 4 or more. Though there is no upper limit in particular, in one embodiment, the carbon number is 10 or less.

The alkyl substitution product of the pyridylnaphthalenediimide is preferably the following compound. Preferably, it is N-pyridyl-N'-alkyl-naphthalenediimide, and the carbon number of the alkyl group is 3 to 9.

### (Supramolecular complex)

In the present specification, a supramolecular complex refers to a complex having numerous porphyrins and numerous metal atoms in a single molecule.

As one example of a preferable supramolecular complex, the formula of a supramolecular complex of zinc (II) porphyrin dendrimer-PyNIm supramolecular complex [D(ZnP-PyNIm)₁₆] is shown below (Ar = 3,5-di-t-butylphenyl). In the formula, the pyridylnaphthalenediimide is N-pyridyl-N'-hexyl-pyridylnaphthalenediimide.

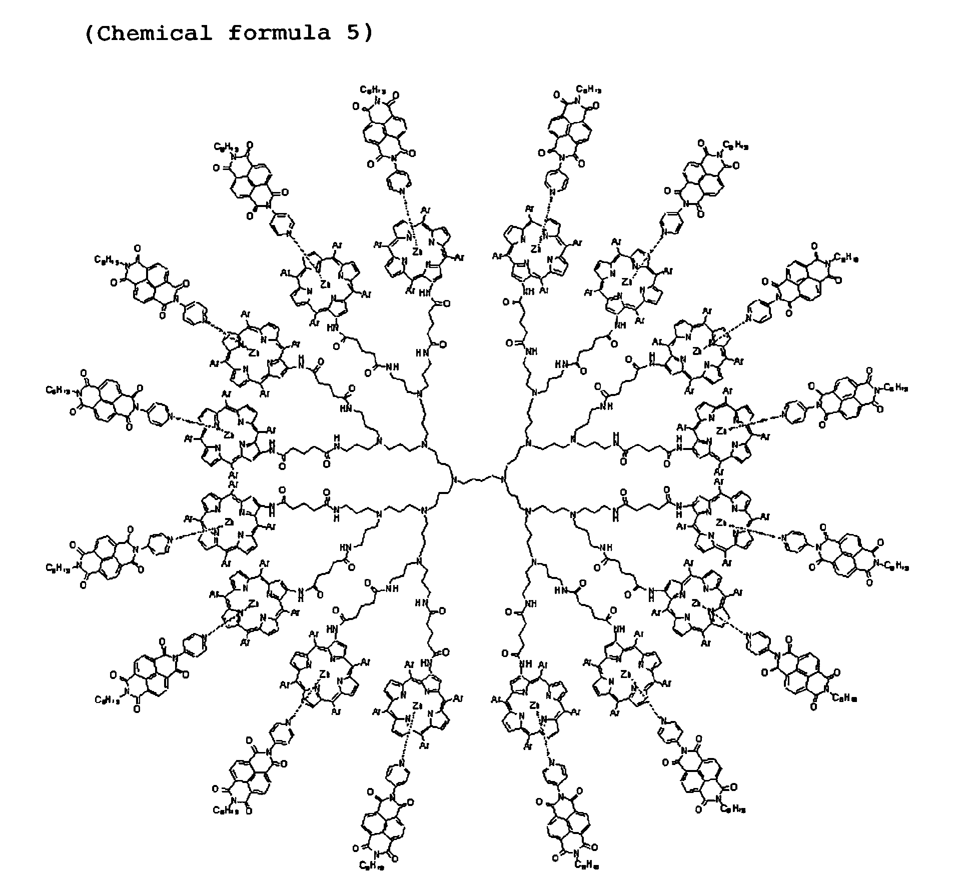

In the present invention, it is preferable that pyridylnaphthalenediimide is axially coordinated to the metal atom of all the porphyrins in the porphyrin dendrimer; however, the object of the present invention will not be greatly deteriorated even if there are some porphyrins to which pyridylnaphthalenediimide is not bonded, as required.

Preferably, pyridylnaphthalenediimide is bonded to 30 % or more of the total number of the metal atoms of the porphyrins in the porphyrin dendrimer. More preferably, pyridylnaphthalenediimide is bonded to 50 % or more thereof. Still more preferably, pyridylnaphthalenediimide is bonded to 70 % or more thereof. Especially preferably, pyridylnaphthalenediimide is bonded to 90 % or more thereof.

The present invention is characterized in that a plurality of pyridylnaphthalenediimides are axially coordinated to one molecule. Therefore, as the absolute number of the axially coordinated pyridylnaphthalenediimides, in one supramolecule, preferably five or more pyridylnaphthalenediimides are bonded; more preferably eight or more pyridylnaphthalenediimides are bonded; still more preferably twelve or more pyridylnaphthalenediimides are bonded; and especially preferably fifteen or more pyridylnaphthalenediimides are bonded.

### (Solvent)

The solvent used in the method of the present invention is not particularly limited as long as the solvent can dissolve the porphyrin dendrimer serving as a raw material and the substituted or non-substituted pyridylnaphthalenediimide. The solvent is preferably benzonitrile.

### (Formation of a complex of porphyrin dendrimer and pyridylnaphthalenediimide)

By mixing the porphyrin dendrimer and the substituted or non-substituted pyridylnaphthalenediimide in a suitable solvent, axial coordination bond can be formed between the metal atoms of the porphyrin dendrimer and the nitrogen of the pyridyl group of pyridylnaphthalenediimide, whereby a supramolecular complex can be formed.

The concentration of pyridylnaphthalenediimide at the time of mixing is not particularly limited; however, the concentration is preferably 1 x 10⁻⁵ M or higher, more preferably 1 x 10⁻⁴ M or higher, especially preferably 1 x 10⁻³ M or higher. Further, the concentration is preferably 1 x 10⁻¹ M or lower, more preferably 5 x 10⁻² M or lower, especially preferably 1 x 10⁻² M or lower. When the concentration is too low, the yield of the obtained supramolecular complex will be small. When the concentration is too high, it will likely be difficult to dissolve the pyridylnaphthalenediimide.

The concentration of the porphyrin dendrimer at the time of mixing is not particularly limited; however, the concentration is preferably 1 x 10⁻⁹ M or higher, more preferably 1 x 10⁻⁸ M or higher, especially preferably 1 x 10⁻⁷ M or higher. Further, the concentration is preferably 1 x 10⁻⁴ M or lower, more preferably 1 x 10⁻⁵ M or lower, especially preferably 1 x 10⁻⁶ M or lower. When the concentration is too low, the yield of the obtained supramolecular complex will be small. When the concentration is too high, it will likely be difficult to dissolve the porphyrin dendrimer.

The molar ratio of mixing the porphyrin dendrimer and the pyridylnaphthalenediimide is not particularly limited. They can be mixed at an arbitrary molar ratio. It is preferable that pyridylnaphthalenediimide is largely excessive relative to the porphyrin dendrimer. With respect to 1 mol of the porphyrin dendrimer, the pyridylnaphthalenediimide is preferably 1 x 10 mol to 1 x 10⁵ mol, more preferably 1 x 10² mol to 1 x 10⁴ mol, still more preferably 1 x 10³ mol to 5 x 10³ mol.

### (Photoinduced electron transfer reaction of supramolecular complex)

It is possible to use the supramolecular complex of the present invention for performing a photoinduced electron transfer reaction. The photoinduced electron transfer reaction of a supramolecular complex can be confirmed by the method described in the later-described Examples or the like.

### (Light energy conversion material)

In the present specification, a light energy conversion material refers to a material that converts light into electric energy. The light energy conversion material is usable in devices such as a solar cell and a photosensor. By using the material of the present invention in a known production method with respect to these devices, a device having an excellent performance can be produced.

### (Material for an artificial photosynthetic reaction center)

The supramolecular complex of the present invention can be used as a material for an artificial photosynthetic reaction center. Conventionally, a known material for an artificial photosynthetic reaction center is obtained by covalently bonding an electron donor molecule and an electron acceptor molecule. Similarly to the conventional material used for an artificial photosynthetic reaction center, the supramolecular complex of the present invention can be used as an artificial photosynthetic reaction center.

### (Hydrogen-producing photocatalyst)

The supramolecular complex of the present invention can be used for a hydrogen-producing photocatalyst. Conventionally, a known hydrogen-producing photocatalyst is obtained by combining a water-reducing catalyst and a porphyrin derivative. By using the supramolecular complex of the present invention in place of the porphyrin derivative of the conventional catalyst, a hydrogen-producing photocatalyst of the present invention can be obtained. For example, by laminating a platinum catalyst on a substrate such as glass and by further laminating a supramolecular complex thereon, a hydrogen-producing catalyst can be formed.

### (Hydrogen synthesis method)

The supramolecular complex of the present invention can be used in a hydrogen synthesis method. For example, by irradiating water with light in the presence of a molecule serving as an electron source and a hydrogen-producing photocatalyst containing a supramolecular complex and a platinum catalyst, water can be reduced to generate hydrogen. The molecule serving as an electron source can be an arbitrary compound, such as molecule that can give electrons to an excited state of a photocatalyst. Specifically, an analog of dihydronicotinamide adenine dinucleotide (NADH), which is an important electron source in a living body, for example, 1-benzyl-1,4-dihydronicotinamide and the like can be used.

### (Device)

The supramolecular complex of the present invention can be effectively used as a material for a conventionally known device that converts light into electric current. For example, it can be used as a material for a photoelectric conversion device. The construction of the device can adopt an arbitrary construction conventionally known in the art. For example, by laminating a supramolecular complex on an electroconductive substrate, a device that converts light into electric current can be obtained.

### EXAMPLES

Hereafter, non-limiting Examples of the present invention will be described.

### (Material)

Polypropyleneimine hexadecaamine dendrimer (generation 3.0), 1,2-dimethoxyethane, N-hydroxysuccinimide, and 1,3-dicyclohexylcarbodiimide were obtained from Aldrich Chemical Company, Inc. Glutaric acid and zinc (II) acetate were obtained from Tokyo Kasei Kogyo Co., Ltd. Chloroform, hexane, dichloromethane, and benzonitrile (PhCN) were purchased from Wako Pure Chemical Ind., Ltd. Benzonitrile and acetonitrile were purified by successive distillation over calcium hydride. By the same procedures as those described in a prior art document, 2-amino-[5, 10, 15, 20-tetrakis(3,5-di-*tert*-butylphenyl)porphyrin and pyridylnaphthalenediimide (PyNIm) were prepared.

### (Synthesis of zinc porphyrin dendrimer [D(ZnP)₁₆])

5-{amino-2-[5,10,15,20-tetrakis(3,5-di-*tert*-butylphenyl )porphyrin]}-5-oxopentanoic acid.
2-amino-[5,10,15,20-tetrakis(3,5-di-*tert*-butylphenyl)po rphyrin (3.99 g, 3.70 mmol) in dry toluene (100mL) was heated at reflux for five hours together with glutaric anhydride (4.22 g, 37mmol). This mixture was left to stand for cooling, and dichloromethane (50 mL) was added. This solution was washed with water (3 x 100 mL), and dried over anhydrous sodium sulfate. The solvent was removed, and the product was purified by column chromatography over silica (Type 9385, methanol/dichloromethane; 1:25) to yield the desired product, 5-{amino-2-[5,10,15,20-tetrakis(3,5-di-*tert*-butylphenyl )porphyrin]}-5-oxopentanoic acid (3.96 g, 3.32 mmol, 90%) as a purple brown microcrystalline solid.

5-{amino-2-[5,10,15,20-tetrakis(3,5-di-*tert*-butylphenyl )porphyrinato]zinc(II)}-5-oxopentanoic acid.
5-{amino-2-[5,10,15,20-tetrakis(3,5-di-*tert*-butylphenyl )porphyrin]}-5-oxopentanoic acid (3.30 mg, 2.77 mmol) and zinc(II) acetate dihydrate (3.63mg, 18.0 mmol) in chloroform (125 mL) and methanol (25 mL), was heated at reflux for one hour. The solvent was removed, chloroform (50 mL) was added and this mixture was washed with water (3 x 150 mL), dried over anhydrous sodium sulfate and filtrated, and the solvent was removed. The residue was purified by column chromatography over silica (Type 9385, MeOH/chloroform; 1:25). The major red band was collected, and the solvent was removed to obtain 5-{amino-2-[5,10,15,20-tetrakis(3,5-di-*tert*-butylphenyl )porphyrinato]zinc(II)}-5-oxopentanoic acid as dark red microcrystal.

5-{amino-2-[5,10,15,20-tetrakis(3,5-di-*tert*-butylphenyl )porphyrinato]zinc(II)}-5-oxopentanoic acid N-hydroxysuccinimide.
5-{amino-2-[5,10,15,20-tetrakis(3,5-di-*tert*-butylphenyl )porphyrinato]zinc(II)}-5-oxopentanoic acid (496 mg, 0.39 mmol) in dry 1,2-dimethoxyethane (10 mL) was cooled on ice. N-hydroxysuccinimide (102 mg, 0.89 mmol) and 1,3-dicyclohexylcarbodiimide (DCC) (171 mg, 0.83 mmol) were added, and this reaction mixture was cooled on ice and stirred for five hours under atmospheric pressure of nitrogen. This mixture was filtered through a plug of silica, and the residue was purified by column chromatography over silica (Type 9385, chloroform). The solvent was removed to yield 5-{amino-2-[5,10,15,20-tetrakis(3,5-di-tert-butylphenyl )porphyrinato]zinc(II)}-5-oxopentanoic acid N-hydroxysuccinimide (259 mg, 0.19 mmol , 49%) as a purple crude product.

D(ZnP)₁₆.
5-{amino-2-[5,10,15,20-tetrakis(3,5-di-*tert*-butylphenyl )porphyrinato]zinc(II)}-5-oxopentanoic acid N-hydroxysuccinimide (259 mg, 0.19 mmol) and polypropyleneimine dendrimer G-3 (5 mg, 0.0030 mmol) were dissolved in dichloromethane (3 mL) and triethylamine (20 µL). This reaction mixture was stirred for 1.5 hours under nitrogen in a dark place. This solution was then diluted with dichloromethane (30 mL), washed with water (3 x 30 mL), and dried over anhydrous sodium sulfate, and the solvent was removed. The residue was dissolved in a minimum amount of toluene, and ran through a size exclusion column (20 cm, bio-beads S-X1 in toluene). The solvent was removed to yield D(ZnP)₁₆ as a purple solid.

### (Photophysical measurement)

UV-visible spectra were obtained at 298K on a Shimadzu UV-3100PC spectrometer or Hewlett Packard 8452A diode array spectrophotometer. Corrected fluorescence spectra were taken using a Shimadzu spectrofluorophotometer (RF-5000PC). The solutions were deaerated by argon purging for 15 minutes prior to the measurements. The measurements of transient absorption spectra in various solvents were performed using a Nd:YAG laser (GCR-130, Quanta-Ray) of 431 nm. A pulsed xenon flash lamp (XF-80-60, Tokyo Instruments) was used for the probe beam. The output was recorded with a digitizing oscilloscope (HP 54510B, 300 MHz).

### (Results and considerations)

It has been confirmed that pyridylnaphthalenediimide (PyNIm) and dendrimer zinc (II) porphyrin [D(ZnP)₁₆] in benzonitrile (PhCN) forms a supramolecular complex, and that the supramolecular complex [D(ZnP-PyNIm)₁₆] contains multiple (16) reaction centers.

Specifically, zinc(II) porphyrin dendrimer [D(ZnP)₁₆] was synthesized by terminal functionalization of corresponding polypropyleneimine with activated zinc porphyrin using the same method for the synthesis of free-base porphyrin dendrimers and characterized by ¹³C NMR spectrum and MALDI-TOF MS spectrum (Hasobe, T.; Kashiwagi, Y.; Absalom, M. A.; Hosomizu, K.; Crossley, M. J.; Imahori, H.; Kamat, P. V.; Fukuzumi, S. Adv. Mater. 2004, in press).

Pyridyl-naphthalenediimide (PyNIm) was synthesized by the condensation of 4-aminopyridine with naphthalenediimide and characterized by ¹H NMR spectrum, FAB MS spectrum, and MALDI-TOF MS spectrum.

The UV-visible spectra of D(ZnP)₁₆ at 298K in benzonitrile (PhCN) were changed upon addition of PyNIm, where the Soret band and the Q band are red-shifted with isosbestic points (Figure 2). The absorbance change exhibits a saturation behavior with increasing PyNIm concentration (closed circles in Figure 3). This indicates that PyNIm forms a 1:1 complex with D(ZnP)₁₆. It is well known that such nitrogenous bases readily bind to zinc(II) porphyrins with a 1:1 stoichiometry (Sanders, J. K. M.; Banpos, N.; Clude-Watson, Z.; Darling, S. L.; Hawaley, J. C.; Kim, H. -J.; Mak, C. C.; Webb, S. J. In The Porphyrin Handbook; Kadish, K. M., Smith, K. M. , Guilard, R., Eds.; Academic Press: San Diego, 2000; Vol. 3, pp. 1-48).

Therefore, the obtained complex has been confirmed to be the following compound.

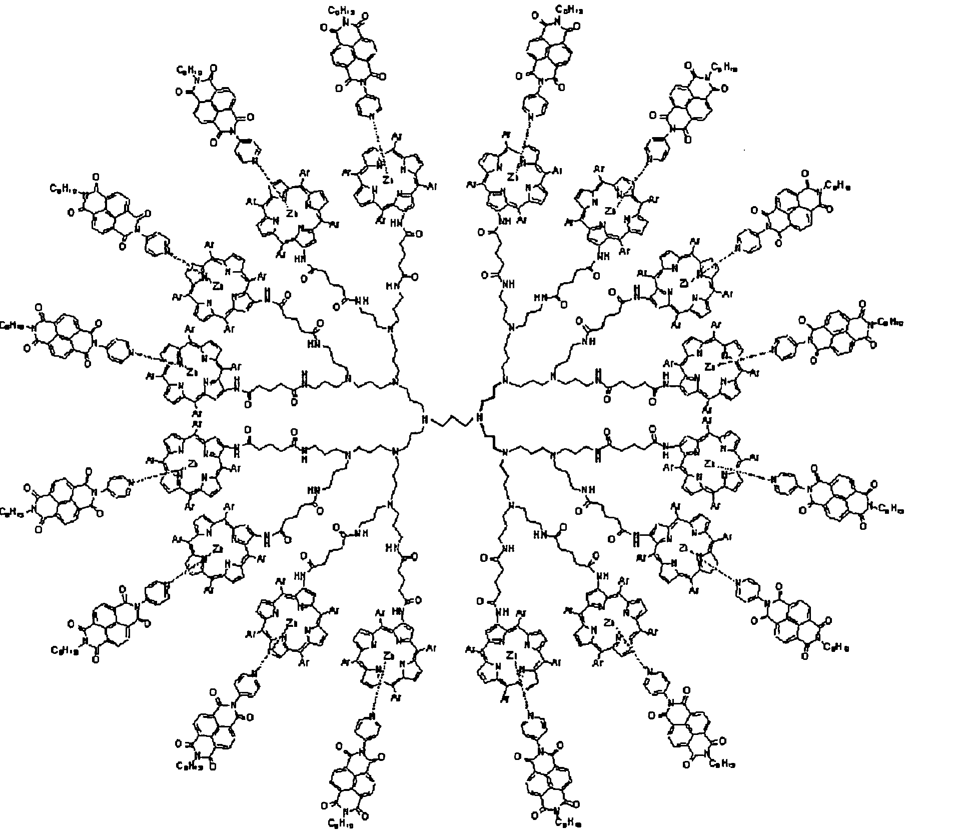

Please note that in the above structural formula, the substituent Ar at 5-position, 10-position, 15-position, and 20-position of each porphyrin is 3,5-di-*tert*-butylphenyl, and the alkyl group bonded to the nitrogen on the outside of naphthalenediimide is straight-chain -C₆H₁₃.

Figure 1 shows a graph of the UV-vis spectral changes of D(ZnP)₁₆ (2.9 x 10⁻⁶ M based on the number of porphyrin units) in the presence of various concentrations of PyNIm (0 to 4.9 mM) in PhCN at 298K.

Figure 2 shows a fluorescence spectra of D(ZnP)₁₆ (2.9 x 10⁻⁶ M) at 298K in deaerated PhCN in the presence of PyNIm of various concentrations (0 to 4.9 mM).

By photoexcitation of the Soret band of D(ZnP)₁₆ at 430 nm in PhCN, fluorescences of λₘₐₓ = 612 nm and 652 nmwere generated. When PyNIm was added to a PhCN solution of D(ZnP)₁₆, a significant change occurred in the fluorescence spectrum of D(ZnP)₁₆ (Figure 2). The magnitude of the fluorescence intensity change of D(ZnP)₁₆ increases in accordance with the increase in the PyNIm concentration and becomes constant, as shown in Figure 3 (open circles). Such fluorescence intensity change is ascribed to photoinduced electron transfer from the singlet excited state of D(ZnP)₁₆ to PyNIm in the supramolecular dendrimer complex. The (A - A₀) / (A₈ - A₀) value in the UV-vis spectral change (closed circles in Figure 3) corresponds to the mol ratio of the ZnP moieties which binds with PyNIm to all the ZnP moieties of D(ZnP)₁₆, and the formation constant K was determined from this value. The K value thus determined decreased with increasing concentration of PyNIm, as shown in Figure 4 (closed circles). Such a decrease in the K value with increasing concentration of PyNIm may be attributed to the steric hindrance of adjacent PyNIm molecules which delay the binding of PyNIm to the zinc ion of the ZnP moiety of D(ZnP)₁₆.

Figure 3 shows plots of (A - A₀) / (A₈ - A₀) (closed circles) and (I₀ - I) / (I₀ - I₈) (open circles) relative to [PyNIm]. (A₈ - A₀) and (I₀ - I₈) are estimated by extrapolation of [PyNIm] dependence of spectral changes (fluorescence intensity at 659 nm and absorbance at 450 nm). (A₈ - A₀) = 0.195, (I₀ - I₈) = 324.

Figure 4 shows dependence of K of supramolecular complex formation between the ZnP moieties of D(ZnP)₁₆ with PyNIm at 298K in PhCN, relative to the concentration of PyNIm. The closed circles and the open circles correspond to the K values determined from the plots of (A - A₀) / (A₈ - A₀) and (I₀ - I) / (I₀ - I₈), respectively.

The (I₀ - I) / (I₀ - I₈) value in the fluorescence spectrum change (open circles in Fig. 3) can also correspond to the molar ratio of the ZnP moieties bonded to PyNIm to all the ZnP moieties in D(ZnP)₁₆. However, this (I₀ - I) / (I₀ - I₈) value is considerably larger than the (A - A₀) / (A₈ - A₀) value at the same PyNIm concentration. This shows that excitation energy migration between porphyrin units occurred efficiently. In this case, the fluorescence of unbound ZnP moieties was eventually quenched by the PyNIm bonded to different ZnP moieties that had not been initially subjected to photoexcitation, as shown in Figure 5.

Figure 5 shows a schematic presentation of energy migration and electron transfer of the singlet excited state and electron transfer in the supramolecular D(ZnP-PyNIm)₁₆ complex. In this figure, EN refers to energy migration.

In order to determine the free energy change of photoinduced electron transfer (ΔG⁰_{ET}), the redox potentials of D(ZnP)₁₆, ZnTBPP, and PyNIm were determined by the cyclic voltammetry measurements in PhCN, as listed in Table 1. The free energy change of photoinduced electron transfer from the singlet excited state of D(ZnP)₁₆ to PyNIm in PhCN was determined to be -0.68 eV from the one-electron oxidation potential in PhCN, the excitation energy (S₁ = 2.05 eV) of 16(ZnP), and the one-electron reduction potential of PyNIm in PhCN. These redox potentials were determined by the cyclic voltammetry in PhCN. By judging from the exothermic photoinduced electron transfer process, it is considered that the fluorescence quenching may result from photoinduced electron transfer from the singlet excited state of D(ZnP)₁₆ to PyNIm in the supramolecular dendrimer complex.

The occurrence of this photoinduced electron transfer in the supramolecular dendrimer complex was confirmed by transient absorption spectra of D(ZnP-PyNIm)₁₆ complex measured in PhCN using nanosecond laser flash photolysis, as shown in Figure 6. The transient absorption bands observed in Figure 6 agree with those of superposition of the absorption bands due to ZnP^{.+} (λₘₐₓ = 640 nm) produced by the one-electron oxidation of D(ZnP)₁₆ with Ru(bpy)₃³⁺ (bpy = 2,2'-bipyridine) and the absorption bands of PyNIm radical anion (λₘₐₓ = 480, 608, 702, and 779 nm) produced by the one-electron reduction of PyNIm using tetramethylsemiquinone radical anion in PhCN (Fukuzumi, S.; Nakanishi, I.; Suenobu, T.; Kadish, K. M. J. Am. Chem. Soc. 1999, 121, 3468). Thus, the transient absorption spectrum in Figure 6 clearly indicates formation of the CS state of the D(ZnP-PyNIm)₁₆ supramolecular complex by photoinduced electron transfer from the singlet excited state of D(ZnP)₁₆ to PyNIm in the supramolecular dendrimer complex. When PhCN was replaced by benzene that is much less polar than PhCN, no CS state was observed, instead only the triplet excited state of D(ZnP)₁₆ was detected as transient absorption spectra.

Table 1. Redox Potentials of D(ZnP)₁₆, ZnTBPP , and PyNIm in PhCN

| compound | *E*⁰ₒₓ vs SCE | *E*⁰_{red} vs SCE |
|---|---|---|
| 16(ZnP) | 0.81 | - |
| ZnTBPP | 0.81 | - |
| PyNIm | - | -0.56 |

The CS state detected in Figure 6 decayed obeying clear definite first-order kinetics (Figure 7) : the first-order plots of different initial CS concentrations afforded linear correlations with the same slope (inset of Figure 7). Thus, the decay process is ascribed to back electron transfer in the supramolecular dendrimer complex rather than intermolecular back electron transfer between ZnP^{.+} and PyNIm^{.-} produced by intermolecular photoinduced electron transfer. The lifetime of the CS state of D(ZnP-PyNIm)₁₆ supramolecular complex was determined to be 830 µs at 298K. The quantum yield of the charge separation was determined to be 0.88 from the comparison of the absorbance of the porphyrin triplet-triplet absorption of ZnTPP in solution (ε₄₇₀ = 74000 M⁻¹cm⁻¹)¹⁵ and the absorption of the CS state (ε₆₄₀ = 12000 M⁻¹cm⁻¹)¹⁶.

As a result, a system having multiple photosynthetic reaction centers has been successfully constructed by using supramolecular complexes formed between zinc porphyrin dendrimers and pyridylnaphthalenediimide. The excitation energy migration occurred between porphyrin units. For this reason, the CS state of the supramolecular dendrimer complex produced upon laser excitation had a long lifetime (830 µs) at 298K in PhCN.

Figure 6 shows a transient absorption spectrum of D(ZnP)₁₆ (2.9 x 10⁻⁶ M) at 298K in deaerated PhCN in the presence of PyNIm (4.9 x 10⁻³ M), as measured at 20 µs after the laser excitation at 431 nm.

Figure 7 shows a time profile of absorption at 620 nm due to the CS state obtained by nanosecond flash photolysis with different laser power (10mJ, 2.2mJ, and 0.5 mJ, respectively). The inset shows first-order plot for the decay of absorbance at620 nm (10 mJ, crosses; 2.2 mJ, closed squares; 0.5 mJ, closed circles).

From the above results, a light energy conversion system having numerous artificial photosynthetic reaction centers, exhibiting high light-harvesting capability and charge separation capability, has been constructed by using a zinc porphyrin dendrimer having numerous porphyrins for enhancing the light-harvesting capability and forming a supramolecular complex utilizing coordination bond between the zinc porphyrin dendrimer and pyridylnaphthalenediimide. From the ultraviolet-visible absorption and fluorescence spectrum in benzonitrile solution, it was discovered that when pyridylnaphthalenediimide is added to a zinc porphyrin dendrimer, the pyridyl group is axially coordinated to the zinc site, whereby a supramolecular complex having plural photosynthesis reaction center sites is obtained. In this supramolecule, when the zinc porphyrin site is photoexcited, a long-life charge-separated state is generated by photoinduced electron transfer within the supramolecule, and the supramolecule had an extremely long lifetime of 830 micro second.

In the initial process of photosynthesis, photoabsorption and energy migration to the reaction centers occur first in the light-harvesting antenna composite, and charge separation is carried out at the reaction centers by utilizing the obtained photoenergy. In the present invention, a huge photosynthesis model molecule with a combination of energy migration and charge separation can be constructed with ease by using the supramolecule technique.

It was discovered that, by using a zinc porphyrin dendrimer having numerous porphyrins as a light-harvesting antenna model and by using a supramolecule zinc porphyrin·pyridylnaphthalenediimide linkage system as a charge separation model, the pyridyl group is axially coordinated to the zinc site, thus obtaining a supramolecule having plural reaction center model molecules. It was discovered that, when the zinc porphyrin site is photoexcited in this supramolecule, a long-life charge-separated state is generated by photoinduced electron transfer within the supramolecule. It was also discovered that, when a zinc porphyrin-modified gold cluster is used as a light-harvesting antenna model, a similar supramolecule assembly is formed, thus obtaining a long-life charge-separated state.

### INDUSTRIAL APPLICABILITY

The present invention is not only the first example in which a supramolecular complex formed by using coordination bond generates a charge-separated state having an extremely long lifetime by photoexcitation, but is also important as a light energy conversion system having high light-harvesting capability and charge separation capability due to the numerous artificial photosynthetic reaction centers.

The light energy conversion supramolecule of the present invention has numerous artificial photosynthetic reaction centers, thus a high light-harvesting capability and charge separation capability. The light energy conversion supramolecule of the present invention can also be easily developed to a more complex system, so that the present invention can be applied to organic solar cells and photocatalysts as a highly efficient light energy conversion system.

As described above, the present invention has been illustrated using the preferred embodiments of the present invention. However, the present invention should not be construed to be limited to these embodiments. It is understood that the scope of the present invention should be construed solely on the basis of the claims. It is understood that those skilled in the art can carry out an invention within the scope equivalent to the description of the specification, based on the description of the specific preferred embodiments, the description of the present invention and the common technical knowledge. It is understood that the patents, patent applications, and other documents cited in the present specification should be incorporated by reference in the present specification as if the contents thereof are specifically described herein.

## Claims

1. A complex compound containing a porphyrin dendrimer and a substituted or non-substituted pyridylnaphthalenediimide, wherein a metal is bonded to porphyrins in the compound, and a pyridyl group of the pyridylnaphthalenediimide is axially coordinated to the metal.

2. The compound according to claim 1, wherein said metal is zinc.

3. A complex compound represented by the following formula 1:
(Chemical formula 1) R¹(-N(-R²-N(-R³-N(-R⁴-NHCO-R⁵-CONH-R⁶-R⁷)₂)₂)₂)₂
wherein R¹ is alkylene; four R² are independently alkylene; eight R³ are independently alkylene; sixteen R⁴ are independently alkylene; sixteen R⁵ are independently alkylene; sixteen R⁶ are independently a porphyrin having a metal; the metal is bonded to a central part of each porphyrin; sixteen R⁷ are independently a substituted or non-substituted pyridylnaphthalenediimide; and a pyridyl group of the substituted or non-substituted pyridylnaphthalenediimide is axially coordinated to each of the sixteen metal atoms.

4. The compound according to claim 3, wherein said substituted or non-substituted pyridylnaphthalenediimide is N-pyridyl-N'-alkyl-naphthalenediimide, and the alkyl group has a carbon number of 3 to 9.

5. The compound according to claim 3, wherein R¹, four R², eight R³, sixteen R⁴, and sixteen R⁵ are all -C₃H₆-.

6. The compound according to claim 3, wherein said sixteen metals are all zinc.

7. The compound according to claim 3, wherein said sixteen porphyrins each have a phenyl group, an alkyl-substituted phenyl group, or a halogen-substituted phenyl group at 5-position, 10-position, 15-position, and 20-position thereof.

8. The compound according to claim 3, wherein said sixteen porphyrins each have a phenyl group, a 3,5-di-*tert*-butylphenyl group, or a 2,6-dichlorophenyl group at 5-position, 10-position, 15-position, and 20-position thereof.

9. A method of synthesizing the compound of claim 1, comprising a step of mixing said porphyrin dendrimer and a substituted or non-substituted pyridylnaphthalenediimide in a solvent.

10. A method of synthesizing the compound of claim 3, comprising a step of mixing a porphyrin dendrimer of the following formula and a substituted or non-substituted pyridylnaphthalenediimide in a solvent:
(Chemical formula 2) R¹(-N(-R²-N(-R³-N(-R⁴-NHCO-R⁵-CONH-R⁶)₂)₂)₂)₂

11. The method according to claim 10, wherein said solvent is benzonitrile.

12. A material for an artificial photosynthetic reaction center made of the compound according to claim 1.

13. A hydrogen-producing photocatalyst containing the compound according to claim 1 and a platinum catalyst.

14. A method of synthesizing hydrogen, comprising a step of irradiating water with light in the presence of a molecule serving as an electron source and a hydrogen-producing photocatalyst containing the supramolecular complex according to claim 1 and a platinum catalyst.

15. A device for converting light into electric current, wherein the compound according to claim 1 is laminated on an electroconductive substrate.
